# EUROPEAN PATENT APPLICATION

(11) **EP 1 674 131 A1**
(43) Date of publication of application: **28.06.2006**
(21) Application number: 05111334.8
(22) Date of filing: 25.11.2005
(51) Int. Cl.: A61Q 1/00, A45D 40/16, B01J 6/00

(54) **Production of cosmetic products by moulding**

(30) Priority: 03.12.2004 IT MI20042330
(71) Applicant: Intercos S.p.A., 20122 Milano (IT)
(72) Inventor: Avalle, Nadia, Montana sur Sierre, Vallese (CH)
(74) Representative: Mittler, Enrico

(57) **Abstract**

A process and an apparatus for the production of compact powder cosmetic products with various shapes is described, in which a moulding container (13) made of sintered porous material is utilised, which collects a semi-fluid cosmetic composition or slurry, which is subsequently dried and extracted from the container.

## Description

The present invention concerns a process, an apparatus and a moulding container for the production of compact powder cosmetic products with various shapes.

Traditional compact powder cosmetic products are generally produced by pressing the free powder into a metallic container called bottom or godet, or, more rarely, directly but with the same technique, inside the marketable final container.

The scope of the bottom is to contain the pressed product within rigid walls which can protect it from crashes, in addition to allow an easy housing of the product thus made into the fmal container, as for example by gluing the base.

The traditional technology allows to obtain compact products with smooth surfaces and also with relieves or volumes, the last term meaning extended heights of the products.

However the production of such products involves multiple problems, tied to the technology and to the formulas.

In fact, the pressing of a powder is by norm a difficult process because of the high pressures involved and of the tensions that are created between the product and the cloths that are utilised as anti-adherent and the same bottoms.

For example, the cloths that are placed on the surface of the powder in order to prevent its adhesion to the forming moulds have a low elasticity and therefore low tendency to allow to be shaped, so that, in the case of surfaces with relieves or shapes with a certain volume, they will never have very sharp edges or defined angles, but they will always have rounded shapes and very wide angles.

The traditional technology, in addition, allows also to obtain shapes with relief or with volume which can outflow from the bottom, but always with very precise limits, with values which generally cannot exceed a few millimetres in height, usually from 1 millimetre to a maximum of 4-5 millimetres depending on the dimension of the bottom being utilised.

In addition the walls of the volumes, for example in the case of geometric shapes such as cubes, parallelepiped etc., cannot be straight, but, in order to be subject to pressure, these must have a certain inclination, with consequent modification of the same shape.

From an applicative point of view, in addition, the compact powder in the bottoms with traditional systems always has powder and embrittlement characteristics that are manifested both on the products with smooth surfaces and, in an even more noticeable way, on the relieves and volumes.

This is because there are considerable formulation limits to the use of various raw materials which turn out to be incompatible with the pressing process.

The current state of the art in addition allows the possibility to create simple shapes with volume with the extrusion technique, in which the cosmetic product gets mixed with a solvent, for instance water, until a homogenous mixture is obtained. Such mixture is subsequently extruded in the desired shape, for example square, rectangular etc., is then put to dry on trays and is subsequently cut perpendicularly.

The shapes that it is possible to obtain with this technique, however, are always flat on the two cut faces and on the four sides and the body is always cylindrical, and in this way it is not possible to create pieces having different shapes, such as pyramids, prisms, trunks of cone etc.

In addition, the evaporation of the solvent from the product, as it takes place under the open sky without any contact with any support, causes the formation of a harder surfaces layer, which, sometimes, completely hinders the picking up of the product.

It is obvious that last aspect turns out to be strongly penalizing for the use of this technology.

Last, there is currently also the possibility to obtain more in relief cosmetic shapes, but in this case one must resort to the pouring of an enough liquid "slurry" of product into a container, which, if made of not appropriate material, for example paper, is destined to have problems and large shaping limits, as well as definitely high costs.

A first object of the present invention is to provide a process to obtain compact powder cosmetic products with various shapes which overcomes the aforementioned problems of the known procedures.

A second object of the present invention is to provide an apparatus for obtaining compact powder cosmetic products with various shapes thus overcoming the aforementioned problems.

A third object of the present invention is to provide a moulding container which allows to obtain compact powder cosmetic products with various shapes thus overcoming the aforementioned problems.

According to the invention said first object is attained with a process for the production of compact powder cosmetic products with various shapes, comprising the steps of:
- preparation of a semifluid cosmetic composition (slurry),
- pouring of the cosmetic composition in a volumetric dose meter,
- dosage of the cosmetic composition into a moulding container provided with an opportunely shaped cavity;
- drying of the cosmetic composition with consequent obtainment of a final cosmetic product,
- extraction of the final cosmetic product from the container, characterised in that said moulding container is made of a sintered porous material capable to allow the evaporation of the solvent through the walls of the same container.

According to the invention said second object is attained with an apparatus for the production of compact powder cosmetic products with various shapes, comprising a volumetric dose meter fillable with a semifluid cosmetic composition (slurry), a container of porous material provided with an opportunely shaped cavity fillable with said cosmetic composition, and drying means, characterised in that said container is made up of a sintered porous material capable to allow the evaporation of the solvent through the walls of the same container.

According to the invention said third object is attained with a moulding container for the production of compact powder cosmetic products with various shapes, characterised in that it is made up of a sintered porous material capable to allow the evaporation of the solvent through the walls of the same container.

The use of containers made of sintered porous material allows to provide compact powder products, with very high volume shapes and perfectly straight walls, for example cubes, prisms, parallelepipeds, half-spheres, etc, or very high walls, inclined, with shapes such as prisms, pyramids, cones, trunks of cone etc.

The characteristics of the present invention will become evident from the following detailed description of an embodiment thereof which will be illustrated as a non-limiting example in the enclosed drawings, in which:
Figure 1 shows a view in axial section of a sintered porous container according to the present invention;
Figure 2 shows a section view according to the line II-II in Figure 1;
Figure 3 shows a side view of the volumetric dose meter and the sintered porous container utilised in the process according to the present invention;
Figure 4 shows a side view of the oven for the drying stage;
Figure 5 shows a sectioned side view of the sintered porous container turned upside down for the extraction of the final cosmetic product.

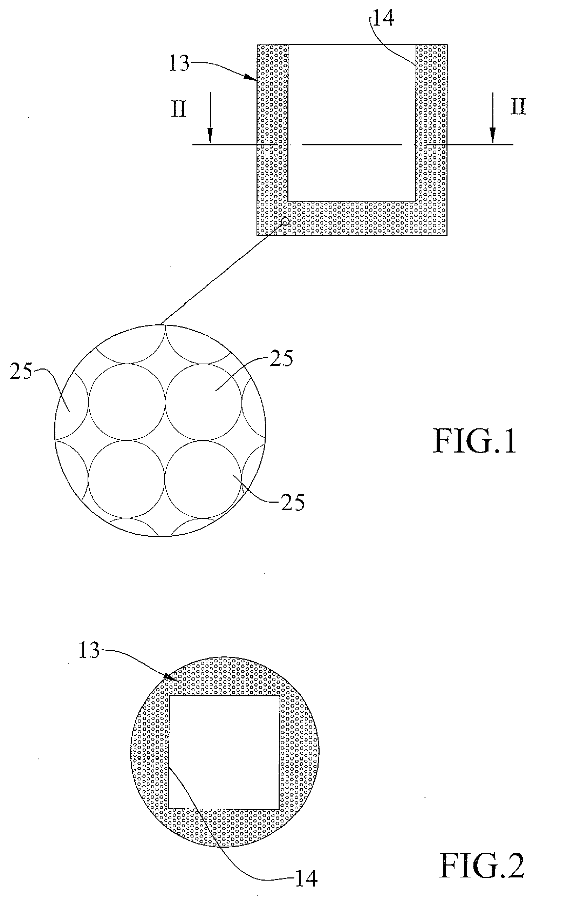

In Figures 1 and 2 a container or sintered porous mould 13, for example metallic, is shown that is provided with an opportunely shaped cavity 14.

The sintered porous metallic material is made up of a compact aggregate of small spheres 25, for example small metal spheres.

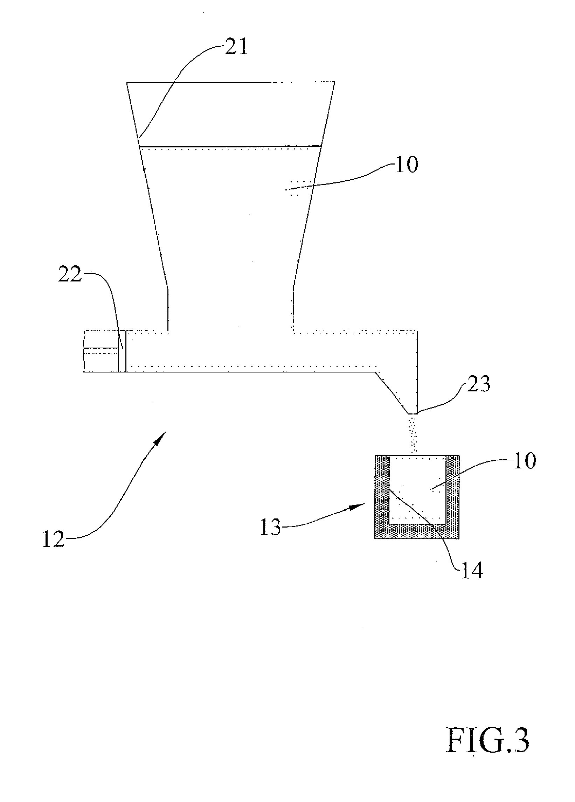

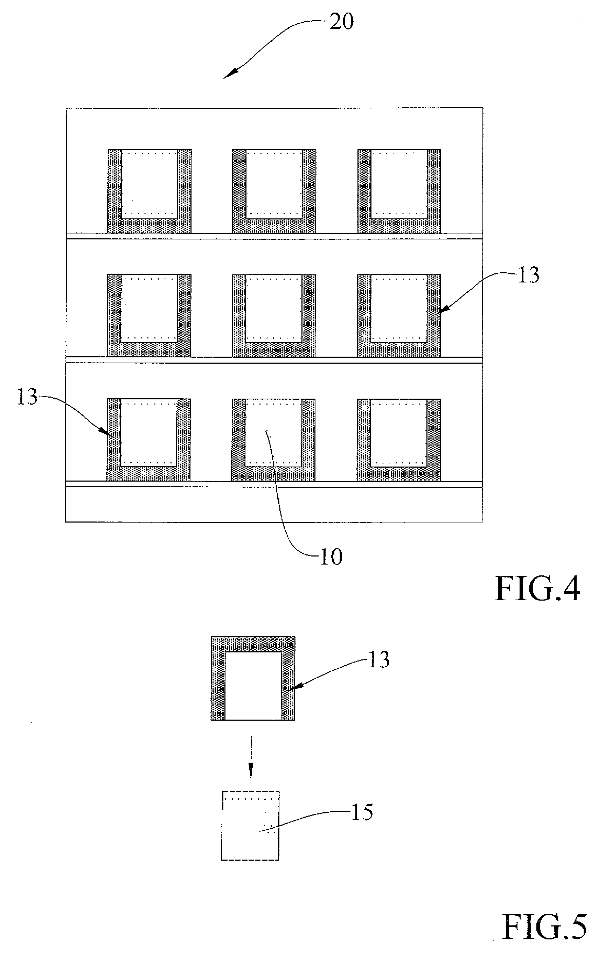

The aforesaid container 13 is usable in a process according to the invention, which utilises an apparatus comprising (figure 3) a volumetric dose meter 12 and the porous sintered mould 13, fillable with a semifluid cosmetic composition or slurry 10 and then insertable into a drying oven 20 (Figure 4).

The volumetric dose meter 12 comprises a collection hopper 21, a dosing piston pump 22 controlled by an external unit not shown in the figures, and a outlet nozzle 23.

In order to obtain the semi-fluid cosmetic composition or slurry 10, a powdery base cosmetic product is dispersed with an opportune solvent (for example water, isopropanole or other) until it reaches the consistency of a dosable slurry/dough.

The slurry 10 thus obtained is then poured (Figure 3) into the hopper 21 of the volumetric dose meter 12 and by means of the dosing piston pump 22, or analogous system, it gets dosed into the sintered porous mould 13.

The dosed cosmetic product 10 is then placed in an oven 20 to dry (figure 4).

The drying temperature and time can vary considerably depending on the solvent being used: from 1-2 hours up to 8-12 hours, from 50°C to 70 °C.

In Figure 5, the final dry cosmetic product 15 gets extracted from the sintered porous mould 13 by turning the same mould 13 upside-down.

The product 15 is now ready to be assembled with the final marketable container.

The procedure above described is utilisable because, being the sintered material made up of a compact aggregate of small metal spheres, generally bronze or stainless steel, with different dimension, for example from 5 to 20 micron, even after heating the structure of this material will remain porous, thus allowing the evaporation of the most volatile products through the porosity, and strongly favouring the separation of the dry product 15 from the sintered porous mould 13 and thus preventing the formation of surface crusts counterproductive for the application of the product 15, which are so frequent in the products 15 obtained through drying with traditional systems.

In addition, the possibility of using a dose measurable slurry, through the use of a solvent, allows the uses of different and more various raw materials which can confer the cosmetic product more performing characteristics.

The slurry 10 dose metered in the sintered mould 13 can also be subject to vacuum pack suction before being dried and immediately extracted from the same mould 13. In this way the processing times are thus reduced and the productive capability increases, thus reducing costs.

The cosmetic product 15 obtained can be inserted directly in the final marketable container without the use of a bottom, and be anchored to it, for example, by insertion of a gluing resin.

Such resin, which can be either transparent or coloured, allows to glue the product 15 inside the container thereby allowing its use, provides protection to the cosmetic product 15 and creates an element of continuity with the container.

It can also be provided that the base of the product 15 is anchored to a grid, which can provide a good base for its gluing to the container, in addition to preventing the outflow of product 15 from the same container.

## Claims

1. Process for the production of compact powder cosmetic products with various shapes, comprising the stages of:
- preparation of a semi-fluid cosmetic composition (slurry) (10),
- pouring of the cosmetic composition (10) into a volumetric dose meter (12),
- dosage of the cosmetic composition (10) inside a moulding container (13) provided with an opportunely shaped cavity (14);
- drying of the cosmetic composition (10) with consequent obtaining of a final cosmetic product (15),
- extraction of the final cosmetic product (15) from the container (13), **characterised in that** said moulding container (13) is made of a sintered porous material (13) capable to allow the evaporation of the solvent through the walls of same container (13).

2. Process according to claim 1, **characterised in that** said cosmetic composition (10) is in the form of dough, made up of a powdery cosmetic product dispersed in an opportune solvent.

3. Process according to claim 1, **characterised in that** said sintered porous container (13) is of metallic material.

4. Process according to claim 1, **characterised in that** said drying (4) takes place in an oven (20).

5. Process according to claim 1, **characterised in that** said drying (4) is preceded by a stage of vacuum pack suction.

6. Process according to claim 1, **characterised in that** the extraction (5) takes place by turning the container (13) upside-down.

7. Apparatus for the production of compact powder cosmetic products with various shapes, comprising a volumetric dose meter (12) fillable with a semi-fluid cosmetic composition (slurry) (10), a porous material container (13) provided with an opportunely shaped cavity (14) fillable with said cosmetic composition (10), and drying means (16), **characterised in that** said container (13) is made up of a sintered porous material capable to allow the evaporation of the solvent through the walls of same container (13).

8. Apparatus according to claim 7, **characterised in that** said volumetric dose meter (12) comprises a collection hopper (18) for the cosmetic composition (10), a dosing pump (19) and an outlet nozzle (20).

9. Apparatus according to claim 7, **characterised in that** said sintered porous container (13) is made of metallic material.

10. Apparatus according to claim 7, **characterised in that** said drying means consist of an oven (20).

11. Moulding container for the production of compact powder cosmetic products with various shapes, **characterized in that** it is made up of a sintered porous material capable to allow the evaporation of the solvent through the walls of the same container (13).

12. Moulding container according to claim 11, **characterised in that** it is made of metallic material.
